# EUROPEAN PATENT APPLICATION

(11) **EP 1 475 097 A2**
(43) Date of publication of application: **10.11.2004**
(21) Application number: 04425330.0
(22) Date of filing: 10.05.2004
(51) Int. Cl.: A61K 35/78, A61P 17/14, A61P 35/00, A61P 17/16, A61P 17/00, A61K 7/42, A61K 7/48

(54) **Dermatological compositions comprising an extract of achyrocline sp (marcela), uses and process for the preparation thereof**

(30) Priority: 08.05.2003 UY 2779903; 18.05.2003 UY 2781403
(71) Applicant: Dajas Mendez, José Federico, 11200 Montevideo (UY); Gonzalez, Margarita, 00183 Roma (IT)
(72) Inventor: Dajas Mendez, Frederico José, Montevideo 1200 (UY); Heinzen, Horacio c/o Facultad de Quimica, 11800 Montevideo (UY)
(74) Representative: Iannone, Carlo Luigi

(57) **Abstract**

The invention relates to dermatological compositions comprising an extract of *Achyrocline sp* (Marcela), *Achyrocline Flaccida* or *Achyrocline Blanca* having antioxidant, cellular protective and scalp tonic properties, due to the action of the components of said extract and therefore they can be advantageously employed in the prevention and treatment of wrinkles, as solar protectors, in the prevention of the skin tumours and treatment of alopecia. The invention relates to a preparation procedure of the extract and compositions containing the same.

## Description

The present invention pertains to dermatological compositions comprising *Achyrocline sp* extract, uses thereof and preparation procedure. More particularly, the invention refers to dermatological compositions comprising an extract of *Achyrocline sp* (Marcela), *Achyrocline Flaccida* or *Achyrocline Blanca* having antioxidant, cellular protective and scalp tonic properties, due to the action of the components of said extract and therefore they can be advantageously employed in the prevention and treatment of wrinkles, as solar protectors, in the prevention of the skin tumours and treatment of alopecia.

The oxidative stress, intended as an imbalance between the reactive oxygen species (ROS) production and the enzymatic and no antioxidant defences, has been identified as the cause of lesion and cellular death in a lot of pathologies such as, for instance, diabetes, cancer, vascular and neurodegenerative pathologies, arterial hypertension, rheumatism (Halliwell, 1990-1999).

Like a last barrier from the external environment, the skin is particularly exposed to the direct action of the environmental pollution agents and subjected to ultraviolet radiation. It is shown clinically and experimentally that the exposure to the ultraviolet radiation generates ROS, which in turn result in cellular lesions, and furthermore are cause, in the last years, of the significant increase of the skin melanoma (Pfahlberg, 2001).

This situation induced an intensive research and development of dermatological preparations having antioxidant properties that protect the skin from the oxidant exposures and/or from the aggressions by allergen or polluting agents. For this purpose, the natural compounds have been identified as preferred resource of antioxidant compounds and have been incorporated in dermatological preparations such as, for instance, creams, lotions, in a progressively increasing percentage.

The nature is an inexhaustible source of new molecular principles. There are more than 250.000 species of plants from every of which results compounds by tens. This results in million molecules of which only a small percentage has been studied for their pharmacological properties.

Ten out of twenty pharmaceutical products belonging to the list of commercial products worldwide have a natural origin.

Natural compounds with antioxidant properties are present in microorganisms as well as in animals and plants, though the latter ones contain a greater variety of mixtures (Potterat. 1997, Romay. 1998, Saija. 1995, Salah. 1995). From plants, metabolites with antioxidant activity have been isolated, principally of flavonoid type, tannins, cumarins, lignans and xanthines.

A flavonoid example is quercetin, which is present in vegetables and fruits, for a human mean daily consumption of 25 mg/person (NTP, 1992). To this flavonoid, anti-microbial effect (EI-Gammal and Mansour, 1986), inhibition capacity of tumour formation for chemical carcinogenesis (Fujiki et al., 1986), and anti-thrombotic properties (Beretz et al., 1981) have been recognised. In addition, antiviral and antiallergic properties (Vrijsen et al., 1988), inhibitory activity of the platelets aggregation and of the thrombotic activation (Gryglewski et al., 1987) have been shown.

Quercetin has shown inhibitory effects for cell growth of several type of human cancer such as the ovarian tumour (Scambia, et al., 1990) and the gastro-intestinal tumour (Yoshida et al., 1992).

The most biological effects of the quercetin and other flavonoidis can be explained on the basis of their antioxidant and free radical quenching activity.

The authors of the present invention now have found that the *Achyrocline sp* extract can be advantageously employed in cosmetic preparations for his antioxidant properties and as cellular protector.

*Achyrocline satureioides* (Marcela), *Achyrocline Flaccida* or *Achyrocline Blanca* and like, extensively consumed in Uruguay in form of infusion, have been never studied for dermatological application. The chemical composition of these plants has elevated concentration of a particular group of flavonoids such as 3 methyl-o-quercetin, quercetin, luteolin and miricetine, that are cytoprotectors. The Marcela, therefore, associated to the antioxidant effect has a marked action of cellular protection.

Furthermore the authors of the present invention have found that the Marcela extract has an amazing capillary tonic effect and therefore can be advantageously employed in the prevention and care of alopecia.

The skin is the largest organ of the human body. Among its functions, the most remarkable ones are the maintenance of the bodily temperature, role of physical support, excretion of reject substances, generation of vitamin D, pigmentation, sensorial functions, protection and the immunity.

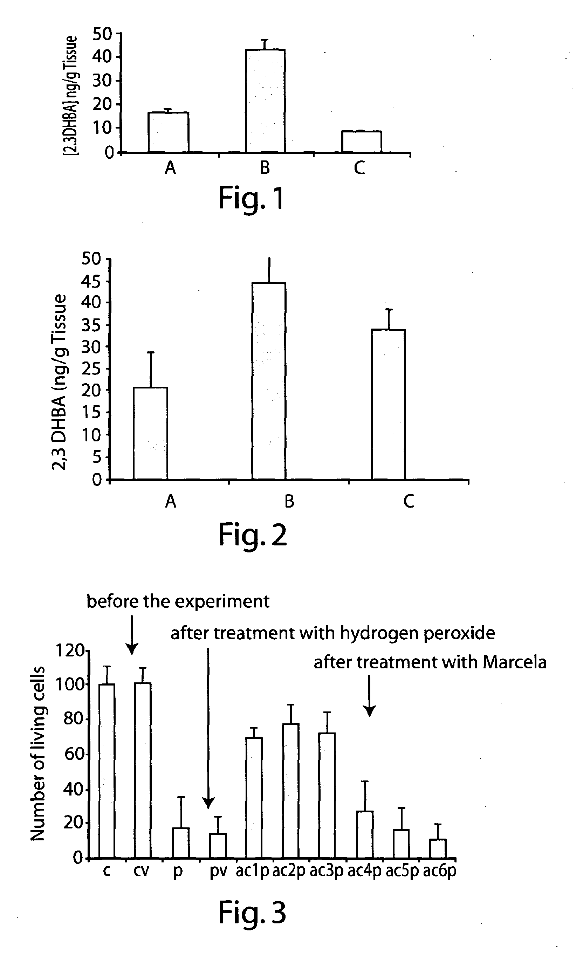

The most important role of the hair, as far as it is concerned, consists of avoiding the losses of heat so far as it is able to retain the air surrounding the skin by forming a limited layer of air (Fig. 1). In addition, the down can provide an indication of the secondary sexual characters which go from the lion's mane to the human beard. The fibre of the hair forms a barrier that helps the protection of epidermis from the damages due to ultraviolet radiation. Specialised hairs such as eyelashes and eyebrows protect the ocular globe from dust and liquids. Nasal hairs play an important role in the treatment of particles contained in the air before they can damage the lungs and they increase the evaporation surface of the sweat beginning from the apocrine glands.

The total number of hair follicles (HF) in the adult is esteemed to be of 5 million, of which one million on the head, of which only 100 thousand cover the scalp (Szabo, 1958). The only regions that do not contain HFs are the palms and soles. HFs can be recognised as a kind of separate entity within the skin, whose formation and maintenance are based on the interaction of derma and epidermis components (Fig.2). HF penetrates the layer of the derma of the skin, composed from fibroblasts and collagen of the connective tissue interposed between blood cells, sebaceous glands and sensorial nerves. The region of bulb is situated upon the subcutaneous tissue.

Under the influence of the dermal papilla (DP), the differentiation of the epidermis cells during the anagenesis, namely the phase of hair growth, produces a keratinous hair fibre and related products. The matrix cells lying next to DP are a group of actively proliferating cells, which differentiate by enriching themselves in keratin up to form the cortex (Co) and the cuticle (Cu). Toward the centre of the fibre is located the medulla (Me).

The cells surrounding the fibre form the inner cavity which is divided in three layers: the cuticle (Cu), the Huxley's and Henle's layers (Fig.3).

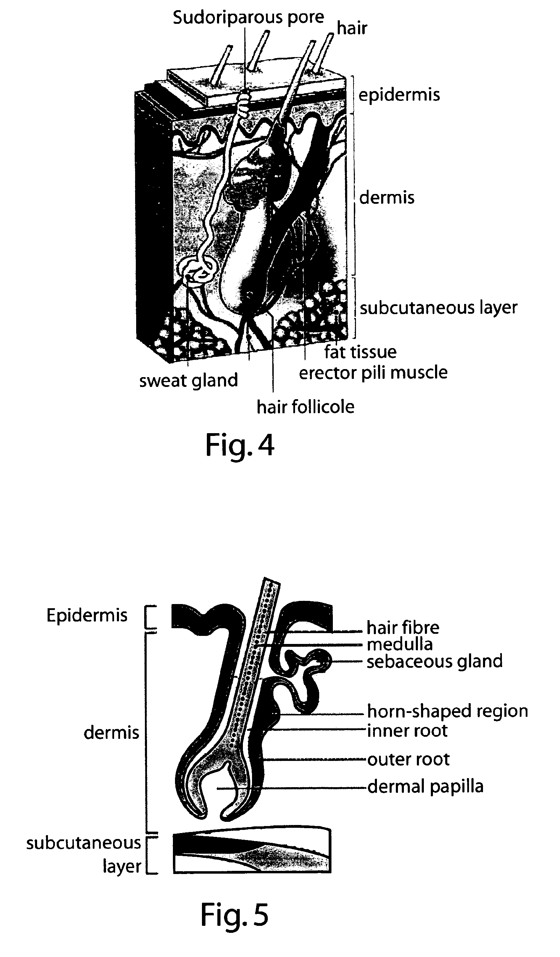

The dermal papilla (DP) has a shape like a pear in the normal follicles (Fig. 5), being formed by a group of very active cells able to induce follicular development and generation of an hair fibre (Oliver, 1966b, Oliver 1967). In proximity of the fibre's root it is possible to find a membrane referred to as basement membrane. The DP is crossed at the base by nervous fibres and blood capillary through narrow cleavages. The greater the DP dimensions and the cells number which contains, the thicker the hair fibre that will be produced by the follicle. The DP cells are very active, and rich of cytoplasm when the follicle contracts producing the fibre. When the follicle does not contract producing the hair, the DP cells lose most of the cytoplasm and they become inactive. The last part of the dermal layer is able to regenerate the DP in absence of the same, infiltrating and transforming the original site with consequent development of the follicle. The DP cells maintain their ability of embryonic regeneration and they are able to induce the growth of new hair fibres in the adult when they are implanted in pre-inactivated follicles.

The capillary fibre is the most important part of the follicle. It is produced by cells that derive from epidermis, referred to as matrix cells, which reproduces and proliferate in order to form the cortical cells. As the aforesaid cells reproduce, their regular and constant production pushes the same toward the surface of the skin. When the cells are moving through the follicle, they begin to differentiate into several particular cellular types. The Co cells widen and keratinize forming layers and, if the follicle contains melanocytes, the melanin is incorporated in the Co. Therefore due to the keratinization the cells end up dying.

Some large follicles show a central layer of few organised cells, forming a tubular structure in the centre of the fibre, referred to as medulla. These arrange themselves as tiles above the cortex and, as the cells of latter, they die through a keratinization process.

The root is the external layer of the root that continues in the epidermis forming an horn-shaped region where the retractable hair muscle is located.

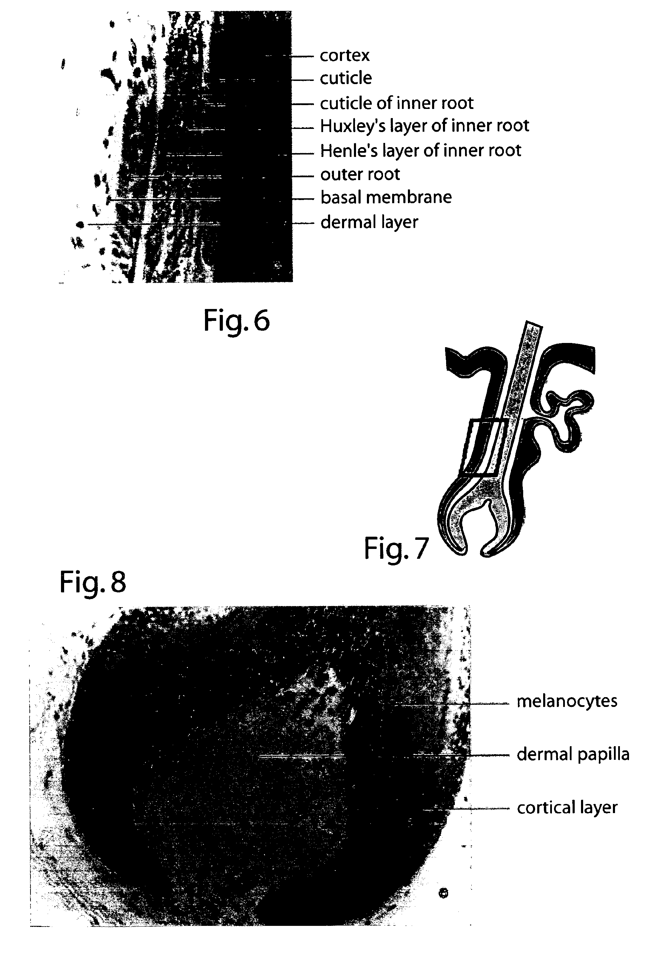

In normal conditions, the hair growth in every follicle is cyclical (Stehn. KS; Paus. R, 1999; Sayto. M et al., 1970; Pelfini. C et al., 1969; Chase. HB et al., 1955). In this cycle there are three principal steps: anagenesis, catagenesis and telogenesis (Fig. 7, 8, 9). The anagenesis is the step of active growth in which the hair is produced. The proanagenesis denotes the beginning of the development, with the synthesis of ADN and ARN in the follicle, which passes quickly from the mesanagenesis to the metanagenesis. In this mature state of proliferation and differentiation, the hair follicle includes 8 concentric layers and the melanogenesis in the pigmented follicles occurs. The anagenesis is followed by the catagenesis, transitory phase of controlled involution of the follicle which finally will enter in telogenesis. The anagenesis is the amplest phase with 90% of the hair follicles of the human scalp in normal conditions in active state of hair growth and the remaining 10% in phase of telogenesis (Kligman, 1988).

The mean rate of growth of the hairy fibre is approximately 0,35 mm/day, but this depends on the place where the follicle is placed, sex and age of the test subject (Pelfini, 1969). The hair length is gene determined. The duration of the hair anagenesis is usually equal to 6-10 years, while for the telogenesis it is equal to 30-90 days, and the catagenesis is esteemed about 14-21 days (Van Scott, 1957; Orfanos, 1990).

In the humans the cycle of every follicle is independent on the near follicle. Normally this cycle of hair production and the following inactivation is characteristic of every follicle, even if some factors can influence and inhibit the hair production and in some cases avoid the physical destruction of the follicle.

The factors that can interfere are the adverse reactions to drugs and cosmetics or the immune response due to radiation, tumours, genetic factors, hormones.

The primary component of the hair is the keratin (Baltenneck. F et al., 2000; Powell, 1997; Lee. LD et al., 1975; Brad bury. JH, 1973). It is a protein consisting of chains of amino acids (aa) which constitutes the cytoskeleton of the epidermis cells.

The regulation of the hair growth in humans depends on some factors such as hormones and cytokines (Stenn. KS and colleagues, 1996; Messenger. AG, 1993). The hair follicles contain receptors for estrogens and androgens, which can modify the FP activity (Ebling. FJ, 1988). Other hormones enclosed therein are the by-products of the thyroid and pituitary glands.

Even if the human follicles seem to realise their cycle in independent way, there are evidences that wounds promote growth and induce anagenesis if the follicle was in telogenesis. Table 1 describes the endogenous substances that influence the growth of the hair (ND: unknown, +: increase; -: diminution).

**Table 1**

| Substance | Target Site | Effect on growth |
|---|---|---|
| fibroblast growth factor (bFGF) | DP | + |
| platelet-derived growth factor (PDGF) | DP | + |
| Transformation beta-factor (TGF-beta) | DP | - |
| Interleukin 1-alpha (IL 1-alpha) | Matrix | - |
| Fibroblast growth factor 5(FGF5) | Matrix | - |
| Epidermal growth factor | Matrix | - |
| Keratinocyte growth factor (KGF) | Matrix | + |
| Insulin-like growth factor I (IGF-I) | Matrix | + |
| Substance P | ND | + |
| Parathyroid hormone | ND | - |
| Estrogens | ND | - |
| Dihydrotestosterone | DP | - |

There are some differences in the nature of the hair fibre in humans of different races (Lee. HJ et al., 2002; Loussouarn. G, 2001; Ahumalo. NP et al., 2000; Sperling. LC, 1999; Eckes. LK, 1985). Therefore different types of hairy fibres are produced by different follicles.

Hippocrates (400 bC) was the first one that used the term alopecia, literally translated as "tiredness disease". This term denotes the phenomenon of hair loss and it results from various causes: of immunological origin, as effect of the administration of drugs, inflammatory and hormonal processes and something else.

Also different types of alopecia exist: aerated, telogen and anagen effluvium, androgenic, inflammatory, etc.

The present invention is referred to the androgenic alopecia (AGA). This type of alopecia is the cause in 95% of the cases of hair loss in the human. It can concern either men or women, even if the men generally manifest greater degrees of loss.

In women the AGA is diffused on the whole scalp; however in men the model of loss follows a characteristic profile, identifiable for the different ethnic groups. Already Hippocrates and Plato observed that the eunuches did not show hair loss.

In 1940 Dr. J. Hamilton deduced that the genetic predisposition in the presence of masculinizing hormones was the factor that caused the AGA. One of these hormones is dihydrotestosterone (DHT), which is achieved from the testosterone through the 5-alpha reductase enzyme (Jamin. C, 1951; Prager. N, 2002; Trueb. RM, 2002; Hoffmann. R, 2002).

DHT contributes to the AGA in the subjects that have a genetic predisposition. It has been observed that the individuals that are afflicted by a deficit of the 5-alpha reductase do not develop AGA. When AGA manifests itself, the large and active follicles of specific areas begin to become more small and less active and they are been reduced in dimensions in every following cycle.

A subject afflicted by AGA not necessarily shows an elevated level of testosterone, since it is sufficient to increase the enzyme activity in order to achieve an increase of the DHT in the follicle.

There are several models in order to classify the evolution of the hair losses process in the man: Hamilton's system (Hamilton. JB, 1951), Norwood's system (Noorwood. OT, 1975), Elbing-Rook's ethnic classification system (Ludwig. E, 1977) Ludwig's classification system (Ludwig. E, 1977), etc.

As it is known in the background art, there are to date several options of AGA pharmacological treatment in the humans.

The treatment with Minoxidil is the most recommended for the therapy of AGA (Khandpur. S et al., 2002). It is required the use for a 3-4 month period in order to observe a result in the re-growth of the follicles and for 6 months in order to esteem, in an appropriated way, its utility in any particular case. It acts increasing the activity of 5-alpha reductase and 17-beta hydroxysteroid dehydrogenase that is the enzyme that converts dihydrotestosterone (DHT) into testosterone. The favourite reaction is that of 17-beta hydroxysteroid dehydrogenase, for which the final result is a DHT diminution (Sato. T et al., 1999).

The effectiveness of the aforesaid product requires the constant use and the daily application and such circumstances along with its cost constitute the disadvantages of use thereof.

The Propecia (finasteride) is a relatively new medicine, it has been administered once a day and to date its use has been experimented only in the humans. It is a 4-aza steroid compound, specific inhibitor of the 5-alpha reductase type II that, as it is known, is the enzyme which metabolizes the testosterone to DHT, avoiding the thinning of the hair fibre.

As for the Minoxidil, the therapy is adopted for a three month period or more in order to achieve the first benefits, the drug must be administered in continuous way and therefore the treatment with this product is expensive.

The retin A has been originally used for the treatment of the acne and skin diseases. Alone or in combination with the Minoxidil it can be effective for the treatment of the AGA and other types of alopecia. Its action mechanism consists in the inhibition of the 5-alpha reductase enzyme.

In some cases it is recommended the use of threonine along with the Minoxidil, in order to increase the object effect considering that the retin A shows some side effects such as increasing sensitivity to the light and altering the pigmentation. If it is used all the same, the application during the night is recommended, in order to decrease the effects due to the solar light.

In order to inhibit the 5 alpha-reductase, as well as to have an effect on AGA, some vitamins are employed. There are studies showing that zinc inhibits the 5 alpha-reductase enzyme and that in combination with the vitamin B6 and D3 (Vegesna V et al., 2002) and azelaic acid inhibits up to 90% the activity of the enzyme.

Finally, Saw Palmetto's extract is administered in form similar to the finasteride. Mainly it decreases the levels of 5 alpha-reductase and secondarily it blocks the ri-captation sites of the cellular membrane required from the cells to absorb DHT (Sinclair RD, 2002). Some studies show that this extract is active on the benign tumour of the prostate, in the similar manner as it acts on AGA, which ability depends on the DHT production.

As above mentioned, the authors of the present invention now have found that the Marcela extract has the ability to inhibit the 5 alpha-reductase enzyme and that therefore a dermatological preparation containing this extract has capillary tonic effect.

Therefore forms a specific object of the present invention a dermatological composition comprising as active ingredient at least an Achyrocline extract chosen from the group consisting of *Achyrocline sp* (Marcela), *Achyrocline Flaccida* or *Achyrocline Blanca* along with one or more adjuvants and/or pharmacologically or cosmetically acceptable excipients. The extract concentration is comprised between 0,02 and 10%, preferably between 0,02 and 5%. Some specific examples of extract concentration are 0,25%, 0,5% and 1 %.

The composition according to the invention can be prepared in form of cream, lotion, shampoo or gel.

The use of the composition as defined above for the cosmetic treatment, particularly, for the prevention and the treatment of the wrinkles and like solar protector, constitutes a further object of the present invention.

In addition, the present invention pertains to the use of the composition as above described for the preparation of a drug having scalp tonic action, for the prevention and care of the alopecia and prevention of the skin tumours.

Furthermore, the use of the extract of *Achyrocline sp* (Marcela), of *Achyrocline Flaccida* or *Achyrocline Blanca* and associations thereof, for the preparation of a dermatological composition like defined above, forms an object of the present invention.

It is a further object of the present invention a procedure for the preparation of an Achyrocline extract comprising the following phases: a) place in a soxhlet 20 ml of 95 % ethanol for 1 gram of dried Achyrocline; b) heat at reflux as far as to get the coloration of the extraction solvent and c) evaporate the solvent.

Finally, the present invention pertains to a method for the preparation of a composition like described above comprising the following steps: a) extraction of Achyrocline in a soxhlet employing 20 ml of 95 % ethanol for 1 gram of dried Achyrocline and heating at reflux as far as to get the coloration of the extraction solvent; b) evaporation of the solvent; c) dissolution of the obtained extract in a mixture of ethanol : water in a ratio from 4:3 to 10:3, preferably equal to 7:3 in a volume equal to 10 ml for 1 gram of extract; d) addition of the solution obtained in the phase c) to a basic preparation for creams, lotions, gel, shampoo in a percentage variable between 0,02 and 10%, preferably between 0,02 and 5%.

The present invention now will be described by way of explanation, but not limitation, according to its preferred embodiments, with particular reference to the figures of the enclosed graphs, in which:
figure 1 shows the tissue levels of 2,3-DHBA produced in A (skin not radiated without cream); B (radiated with UV-B without cream) and C (radiated with UV-B with cream of 1 % Marcela. The data represent the average ± S.D. for p<0,05;
figure 2 shows the tissue levels of 2,3-DHBA produced in A (skin not radiated without cream); B (radiated with UV-B without cream) and C (radiated with UV-B with cream below to 1% Marcela). The data represent the average ± S.D. for p<0,05;
figure 3 shows the analysis of cellular survival in cultures of pc12 cells before treatment with Marcela, along with oxygenated water and after treatment with Marcela (p <0,05);
figure 4 shows the skin and hair structure;
figure 5 shows the diagram of the principal layers diversified in the growth phase (anagenesis) of the hair follicle (HF);
figure 6 shows the photo of an histological sample with the principal layers diversified in the growth phase (anagenesis) of the hair follicle (HF);
figure 7 shows a representative scheme of the portion of the histological sample of the figure 5 in the whole follicle;
figure 8 shows the photo of an histological sample and of the principal layers of the dermal papilla (DP);
figure 9 shows the rabbit back divided in quadrants on which the composition containing *Achyrocline sp* (Marcela) (B) and the vehicle (A) have been applied;
figure 10 shows a scalp of an individual with androgenic alopecia before treatment (A) and three months after treatment (B) with the preparation containing the *Achyrocline sp* (Marcela) extract.

Subsequently the present invention will be described, by way of information, but not limitation, in the examples set forth below.

### EXAMPLE 1: Preparation of the Achyrocline sp (Marcela) extract

Add 20 ml of 95 % ethanol in a soxhlet for 1 gram of dried plant of Achyrocline. Then heat at reflux as far as the solvent shows an increase of coloration.

Subsequently, evaporate the solvent by rotoevaporation and dissolve the obtained residue in a mixture 7:3 ethanol/water (10 ml for 1 gram of extract). Add this solution to a basic cream prepared according to conventional procedure in a percentage variable from 0,02 to 5%.

EXAMPLE 2: *Study on the cellular protecting and antioxidant abilities of the Marcela extract on the skin.*

### Cellular protection

The PC12 line is employed, cultured on plates coated with collagen extracted from rat tail, using as medium RPMI 1640 (85%) supplemented with 5% fetal calf serum and 10% horse inactivated serum, for one hour at 60°C, and 50 U/ml penicillin and 25 ug/ml streptomycin.

Medium was changed every 48 hours and the cells weekly split in a ratio 1:3. Once the confluence is achieved, the cells were incubated at 37°C, in humidified atmosphere containing 5% CO₂.

The PC12 cells have been therefore sub-cultured in 96 well plates, at 20,000 cells/well, beginning the experiment after 24 hours subculture with induction of cellular death by the action of pH, 6 hydroxidopamine (60HDA) or Levodopa.

Different treatments have been carried out in order to provoke a cellular damage: a) 200 uM pH for 30 minutes, b) 150 uM 60HDA for 24 hours or c) 200 uM Levodopa for 24 hours. With these concentrations death of approximately 50% of the cultured cells has been achieved, allowing to appraise the effects of cellular protection or toxicity.

The Marcela extract has been administered starting from a saturated solution and preparing the following dilutions: 1:2, 1:4, 1:8, 1:16, 1:32,1:64, and in different times with respect to application of mixtures that caused the cellular damage (first, during and/or after). By this treatment have been achieved various results.

Native PC12 cells have been cultured in 35 mm plates, previously coated with polyornithine at 0,5×10⁵ cell density, the fetal calf serum and horse inactivated serum has been removed. The Marcela extract was added, and after 24 hours cellular survival has been assessed with respect to controls.

### Microscopy

The evaluation of the cultures has been performed daily with different treatments, especially in the experiments of starvation, and the general morphology and, particularly, the neuritic growth were analysed.

Morphological analysis has been carried out by inverted microscopy through photographic recording and image digitalisation and use of a develop software for the captation and processing of optic microscopy image.

### Cellular survival by MTT

The number of vital cells at the end of the treatment has been quantified through MTT technique (3-(4,5-dimethythiazol-2-yl)-2,5 diphenyl tetrazolium bromide, Denizot et al, 1986), which is cut by the mitochondrial succinic-dehydrogenase enzyme obtaining formazan, a compound detectable by spectrophotometry.

A stock solution of 5 mg/ml MTT was added to the medium, obtaining a final concentration of 0,25 mg/ml. Subsequently, the culture was incubated for 3-4 hours at 37°C, the excess of MTT was removed and the formazan was dissolved with dimethylsulfoxide (DMSO).

Finally, the absorbance has been measured at 570 nm (at 630 nm as reference) which value has been compared with a standard curve for different cell types.

### Statistic analysis

The pertinence of the achieved data in normal populations was verified with the Kolmogorov-Smirnov's test and a variance analysis was effected analysing differences in applying a parametric test like the Student's test.

### Toxicity analysis of local and systemic dermic

In order to obtain toxicity data of the extract object of the invention, 12 white rabbits (6 of every sex) have been used, with healthy skin and with a 2-3 kilos weight (with a weight difference below 10%). 10 animals were placed in individual cages, in an environment with controlled and constant (19-25°C) temperature, 30-70% relative humidity and 12 hour light-obscurity cycle, with water and controlled food (5 g/animal daily).

### Local toxicity

The limit dose employed was 2 g/Kg, applied on 10% of the total surface of the animal. The application area was shaved in a 7-8 cm area, on the left side was applied the placebo, while on the right side was applied the Marcela extract, at a distance of 2 cm inside both quadrants.

The exposure time to the product was 4 hours and the observation occurred after two weeks.

For the product application distilled water has been used as vehicle, at room temperature, and the damage has been appraised in the period from 1 to 16 hours, using the Drake's local erythema scale.

### Systemic toxicity

Previously, the biochemical analysis of blood has been carried out (alkaline phosphatase, albumin, creatinine, total proteins and transaminase) in every animal (blood has been collected before applying the product, at seventh day and at the end of the treatment).

The histopathology has been made on skin, liver, kidneys, spleen and small intestine.

### Determination of the free radicals (hydroxylic radicals)

The concentration of the hydroxylic radicals (OH') produced with the exposure to the ultraviolet radiation B (UV-B) in the quadrants with and without cream so as at the basal level in the non-radiated quadrant was determined.

Creams of 1 % and 0,25% Marcela extract have been used for this assay.

Description of the quadrants: left side A - non-radiated, without cream; side right B - radiated with UV-B, without cream, C - radiated with UV-B with cream.

0,5 ml of sodium salicylate (1 mg/ml) dissolved in saline was injected subcutaneously in the quadrants A, B and C.

The quadrant B has been treated with cream and has been radiated for 1 hour with a dose of 1810 uW/cm².

The cream has been removed with abundant distilled and deionized water, and skin samples in the zone of salicilate injection have been collected.

The skin samples have been weighed and suspended in 500 ml of 1M perchloric acid, 20 ul of DMSO have been added. Subsequently have been sonicated, centrifuged and the supernatant has been directly placed in HPLC for the quantification of levels of the 2,3-dihydroxibenzoic acid (2,3 DHBA), hydroxylated derivative of salicilate.

### Results

### Antioxidant ability

The study has allowed to quantify production inhibition of free radicals achieved by UV irradiation of the skin of rabbits treated with 1 % and 0,25% Marcela extract in form of cream. As can be observed in figure 1, the production of hydroxylic radicals in the normal skin (A) increases significantly during the exposure to the ultraviolet radiation and the levels decrease significantly during the application of the dermocosmetic preparation of Marcela. This is shown in the figure 2 for a lower concentration (below 1 %).

The ability of the cream to inhibit significantly the production of hydroxylic radicals (OH') against the significant increase produced by UV irradiation.

### Cytoprotector ability

As shown in figure 2, the cytoprotector ability of the Achyrocline extract has been verified, in addition to antioxidant ability thereof.

The toxicity studies show normal results during a six months exposure, therefore it is possible to conclude that the described preparation is safe for human use.

### EXAMPLE 3: Study on the capillary tonic activity of the Marcela extract in rabbits

The rabbits have been treated like in the example 2.

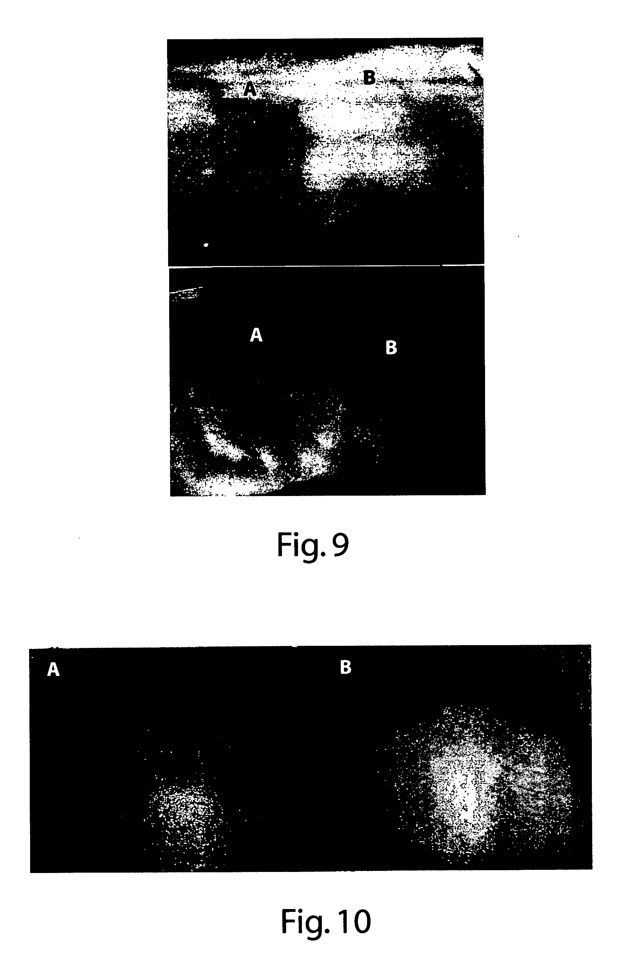

As shown in figure 9, the application of the 1% Marcela extract as a cream or as a lotion causes, a week after the application, a notable increase of hairs in the zone treated with the preparation containing Marcela extract, in contrast with the hair portion covered only with the vehicle that remains without hairs.

### EXAMPLE 4: Study of tonic capillary activity of Marcela extract in cases of alopecia

Lotions with a concentration from 0,5% to 5% of Marcela extract have been prepared.

Healthy male volunteers from 18 to 50 years old that showed AGA have been selected. It is made an initial photo of the head and in profile and a quarterly control photographically documented has been made.

The lotion has been applied in the morning once a day, effecting some circular massages to improve the absorption of the lotion. The concentration of the lotion employed in this study was of 1% of Marcela extract.

Of seven volunteers studied in three months of Marcela extract daily application, all but one showed a clear inhibition of hair losses.

The results are shown in figure 10. As it is possible to see a change of the aspect and the quantity of hair occurs, and in 5 of the 7 cases it is observed an increase of mean intensity with growth of new fibres.

Therefore it is possible to conclude that the preparation containing Marcela in percentage of 1 % has the ability to increase hair growth at experimental level and it has a mean effect on human with full-blown, in some cases advanced, androgenic alopecia.

Bibliography
- Beretz et al. (1981 ); Biochem Pharmacol. 31:3597-3600;
- EI-Gammal, A. et al. (1986), Zentralbl Mikrobioi 141: 561-5;
- Fujiki, H. et al. (1986): inhibition of tumour promotion by flavonoids. In: Cody V. Middleton E. Harborne JB, Eds. Plant flavonoids in biology y medicine: Biochemical, pharmacological, y structure-activity relationships. Pp 429-440 New York. Alan R. Liss
- Gryglewski, R.J. et al. (1987); Biochem. Pharmacol. 36, 317-322.
- Halliwell, B. (1990): How to characterize a biological antioxidant. Free Radic. Res. Commun 9: 1-32. Halliwell, B. Y Gutteridge, J.M.C. (1999): Oxidative stress y ischaemic or traumatic brain injury. In: Free radicals in Biology y Medicine. Third edition (eds) Halliwell, B y Gutteridge, J.M.C. Oxford: University Press pp 733-736.
- Hnasaki, Y. et al., (1994). Free Radic. Biol. Med. 16:6:845-50.
- Ohkawa, H. et al,, (1979), Analytical Biochemistry 95:331-358.
- Pfahlberg, A. Et al. (2001), Br. Dermatol 144(3): 471-475.
- Potterat, O. (1997), Current Organic Chemistry. 1:415-440.
- Re et al., Free Radical Biology & Medicine, 26: 1231-1237.
- Romay, C et al, (1998), Inflamm. Res. 47 (1):36-41. Saija A,. Free Radical Biology & medicine, 19(4): 481-486, 1995.
- Salah, N et al. (1995), Archives of Biochemistry y Biophysics, 232:2:339-346.
- Chase HB. Growth of the hair. Physiol Revs. 1954;34:113-126.
- Yu J, Yu DW, Checkia DM, Freedberg IM, Bertolino AP. Human hair keratins. J Invest Dermatol. 1993 Jul; 101 (1 Suppl):56S-59S.
- Baltenneck F, Bernard, Garson JC, Engstrom P, Riekel C, Leroy F, Franbourg A, Doucet J. Study of the keratinization process in human hair follicle by X-ray microdiffraction. Celi Mol Biol (Noisy-le-grand). 2000 Jul;46(5): 1017-24.
- Powell BC, Rogers GE. The role of keratin proteins and their genes in the growth, structure and properties of hair. EXS 1997,78:59-148.
- Lee LD, Baden HP. Chemistry. And composition of the keratins. Int J Dermatol. 1975Apr;14(3):161-71.
- Bradbury JH. The structure and Chemistry of keratin fibers. Adv Protein Chem. 1973-27:111-211.
- Saitoh M., Uzuka M., Sakamoto M., Human hair Cycle. J. Invest. Dermatol. 1970 Jan;54(1 ):65-81.
- Pelfini C, Cerimele D, Pisanu G. Aging of the skin and hair growth in man. In: Montagna W, Dobson RL (Eds) Hair growth. Pergamon Press Ltd, London, 1969: p153-160.
- Chase MB. The physiology and histochemistry of hair growth. J Soc Cosmetic Chem 1955; 6: 9-14.
- Stenn KS, Paus R. What controls hair follicle cycling? Exp Dermatol. 1999 Aug;8(4):229-33; discussion 233-6.
- Paus R. Principles of hair cycle control. J Dermatol. 1998 Dec;25(12):793-802.
- [17]-Stenn KS, Combates NJ, Eilertsen KJ, Gordon JS, Pardinas JR, Parimoo S, Prouty SM. Hair follicle growth controls. Dermatol Clin. 1996 Oct; 14(4): 543-58.
- [18]-Messenger AG. The control of hair growth: an overview. J Invest Dermatol. 1993 Jul; 101(1 Suppl):4S-9S.
- Ebling FJ. The hair cycle and its regulation. Clin Dermatol. 1988 Oct-Dec;6(4):67-73.
- Lee HJ, Ha SJ, Lee JH, Kim JW, Kim HO, Whiting DA. Hair counts from scalp biopsy specimens in Asians. J Am Acad Dermatol. 2002 Feb;46(2):218-21.
- Loussouarn G. African hair growth parameters. Br J Dermatol. 2001 Aug;145(2):294-7.
- Khumalo NP, Doe PT, Dawber RP, Ferguson DJ. What is normal black African hair? A light and scanning electron-microscopic study. J Am Acad Dermatol. 2000 Nov;43(5 Pt 1):814-20.
- Sperling LC. Hair density in African Americans. Arch Dermatol. 1999 Jun; 135(6):656-8.
- Eckes LK. [Ethnic variations of scalp hair]. Hautarzt. 1985 Jul;36(7):381-5.
- Hamilton JB. Patterned loss of hair in man: types and incidence. Ana N YAcad Dermatol 1951:53,708-28.
- Norwood OT. Male pattern baldness: classification and incidence. South Med J. 1975 Nov;68(11): 1359-65.
- Ludwig E. Classification of the types of androgenetic alopecia (common baldness) occurring in the female sex. Br J Dermatol. 1977Sep;97(3):247

## Claims

1. Dermatological composition comprising as active ingredient at least an Achyrocline extract chosen from the group consisting of *Achyrocline sp* (Marcela), *Achyrocline Flaccida* or *Achyrocline Blanca* along with one or more adjuvants and/or pharmacologically or cosmetically acceptable excipients.

2. Composition according to the claim 1, wherein the extract concentration is comprised between 0,02 and 10%.

3. Composition according to the claim 2, wherein the extract concentration is comprised between 0,02 and 5%.

4. Composition according to anyone of preceding claims, wherein the concentration of the extract is equal to 0,25%.

5. Composition according to anyone of preceding claims, wherein the concentration of the extract is equal to 0,5%.

6. Composition according to anyone of preceding claims, wherein the concentration of the extract is equal to 1 %.

7. Composition according to anyone of preceding claims in a form chosen from the group consisting of cream, lotion, shampoo, gel.

8. Use of the composition as defined in the claims from 1 to 7 for the cosmetic treatment.

9. Use according to the claim 8 for the treatment of the wrinkles.

10. Use according to the claim 8 as solar protector.

11. Use of the composition as defined in the claims from 1 to 7 for the preparation of a drug for the prevention and care of alopecia.

12. Use of the composition as defined in the claims from 1 to 7 for the preparation of a drug having tonic action of the scalp.

13. Use of the composition as defined in the claims from 1 to 7 for the preparation of a drug for the prevention of the skin tumours.

14. Use of an extract of *Achyrocline sp* (Marcela), of *Achyrocline Flaccida,* of *Achyrocline Blanca* or associations thereof for the preparation of a dermatological composition as defined in the claims from 1 to 7.

15. Process for the preparation of an Achyrocline extract comprising the following steps: a) place in a soxhlet 20 ml of 95 % ethanol for 1 gram of dried Achyrocline; b) heat at reflux as far as to get the coloration of the extraction solvent and c) evaporate the solvent.

16. Process for the preparation of a composition as defined in the claims from 1 to 7 comprising the following steps: a) extraction of Achyrocline in a soxhlet employing 20 ml of 95 % ethanol for 1 gram of dried Achyrocline and heating at reflux as far as to get the coloration of the extraction solvent; b) evaporation of the solvent; c) dissolution of the obtained extract in a mixture of ethanol : water in a ratio from 4:3 to 10:3, preferably equal to 7:3 in a volume equal to 10 ml for 1 gram of extract; d) addition of the solution obtained in the step c) to a basic preparation for creams, lotions, gel, shampoo in a percentage variable between 0,02 and 10%, preferably between 0,02 and 5%.
